# EUROPEAN PATENT APPLICATION

(11) **EP 3 255 433 A1**
(43) Date of publication of application: **13.12.2017**
(21) Application number: 16305682.3
(22) Date of filing: 09.06.2016
(51) Int. Cl.: G01N 33/574

(54) **METHODS USING BLM AS A MARKER OF MULTIPLE MYELOMA**

(71) Applicant: Centre Hospitalier Universitaire de Montpellier, 34295 Montpellier (FR); Centre National de la Recherche Scientifique CNRS, 75794 Paris Cedex 16 (FR); UNIVERSITE DE MONTPELLIER, 34090 Montpellier (FR)
(72) Inventor: MOREAUX, Jérôme, 34090 Montpellier (FR); SAPEDE, Elena, 34090 Montpellier (FR); PASERO, Philippe, 34070 Montpellier (FR); CONSTANTINOU, Angelos, 34090 Montpellier (FR)
(74) Representative: Nony

(57) **Abstract**

The present invention relates to the use of an expression level value of BLM in a sample obtained from an individual affected with multiple myeloma for predicting responsiveness of the said individual to a treatment of multiple myeloma.

## Description

### FIELD OF THE INVENTION

The present invention relates to field of prognosis of multiple myeloma by measuring biological markers.

### BACKGROUND OF THE INVENTION

Multiple myeloma is the second form of hematological malignancy. It occurs in increasing frequently with advancing age, with a median age at diagnosis of about 65 years.

Multiple myeloma is the second most common hematological malignancy, although the exact causes of multiple myeloma remain unknown.

It is associated with an uncontrolled clonal proliferation of B-lymphocyte-derived plasma cells within the bone marrow. These myeloma cells replace the normal bone marrow cells, resulting in an abnormal production of cytokines, and in a variety of pathological effects, including for instance anemia, hypercalcemia, immunodeficiency, lytic bone lesions, and renal failure. The presence of a monoclonal immunoglobulin is frequently observed in the serum and/or urine of multiple myeloma patients.

Multiple myeloma is a genetically complex disease that include an aberrant or overexpression of a D-type cyclin gene, cyclin D1 (CCND1) in the case of t(11;14) translocation or gain in 11q13, cyclin D3 (CCND3) in the case of the rare t(6;14) translocation, or cyclin D2 (CCND2) on the background of a translocation involving c-maf (t(14;16)) or MMSET/FGFR3 (t(4;14))(1). In addition to genetic changes, intraclonal heterogeneity of MMCs was recently described as a new level of complexity in MM(Walker et al., 2012, Blood, Vol. 120 (5) : 1077-1086; Magrangeas et al., 2013, Leukemia, Vol. 27 (2) : 473-481; Weston-Bell et al., 2013, Leukemia, Vol. 27 (5) : 1188-1191; Egan et al., 2012, Blood, Vol. 120 (5) : 1060-1066; Keats et al., 2012, Blood, Vol. 120 (5) : 1067-1076; Lohr et al., 2014, Cancer Cell, Vol. 25(1) ; 91-101).

Current treatments for multiple myeloma patients include chemotherapy, preferably associated when possible with autologous stem cell transplantation (ASCT).

Treatment of MM in 2015 (eligible for stem cell transplantation) consists of an induction phase with four monthly courses of high doses of corticosteroid (Dexamethasone) and a proteasome inhibitor, sometimes in association with a cell cycle targeting drug or an immunomodulatory drug, a short term exposure to high dose of melphalan (usually 200 mg per square meter), an alkylating agent followed with autologous stem cell transplantation to rescue hematopoiesis and a maintenance treatment with dexamethasone and an immunomodulatory drug. Approximately one-third of patients with myeloma at diagnosis are older than 75 years and at least 30% are frail, because of the presence of concomitant disease, abnormal laboratory test results and symptoms or signs of disability that may complicate the presentation and management of myeloma. Today, bortezomib-melphalan-prednisone (VMP) and melphalan-prednisone-thalidomide (MPT) are the reference treatments for elderly myeloma patients. Nevertheless, the efficacy of these regimens was less evident in patients aged 75 years or over. These findings raise the question of whether a lower dose intensity treatment with two-drug combinations may improve tolerability, preserving efficacy, in very elderly and frail patients, and thus should be preferred to three-drug combinations.

Survival of patients with multiple myeloma is highly heterogeneous from periods of few weeks to more than ten years. This variability derives from heterogeneity in both tumor and host factors.

Several factors correlated with survival duration in multiple myeloma have been identified, including in particular serum levels of hemoglobin, calcium, creatinine, B2- microglobulin (sβ2M), albumin, C-reactive protein (CRP), platelets count, proliferative activity of bone marrow plasma cells, and deletion of chromosome arm 13q. Subsequently, various combinations of prognostic factors have been suggested for staging classification of myeloma patients. A staging system for multiple myeloma has been developed through an international collaboration between several teams (GREIPP et al., J Clin Oncol, 23, 3412-20, 2005). A combination of serum β2M and serum albumin was retained as providing the simplest, most powerful and reproducible three-stage classification. This International Staging System (ISS) consists of the following stages: stage I, serum β2M less than 3.5 mg/L plus serum albumin >3.5 g/dL (median survival, 62 months); stage II, neither stage I nor III (median survival, 44 months); and stage III, Sβ2M >5.5 mg/L (median survival, 29 months).

It is thus important to identify factors associated with prognosis, in order to better predict disease outcome, and optimize patient treatment.

To diagnose and follow up minimal residual disease in MM, the expression of some markers have already been evaluated in the art by multiparametric flow cytometry (MFC) at protein level. CD19, CD20, CD27, CD28, CD56 and CD117 have been described in the art to discriminate BMPC from MMC (Rawstron et al., 2008, Haematologica, Vol. 91 : 1577-1578). The expression of these CD markers is not homogeneous on all MMC, on one hand all markers do not present aberrant expression and on the other hand expression level of each CD marker changes according to the sample (Gupta et al., 2009, Am. J Clin Pathol, Vol. 132 : 728-732; Raja et al., 2010, Br J Haematol, Vol. 149 : 344-351; Seegmiller et al., 2007, Am J Clin Pathol, Vol. 127 : 176-181; Tembhare et al., 2014, Leuk Res, Vol. 38(3): 371-376).

Indeed, MM is still an incurable disease in the majority of the cases, even if some patients achieve a complete remission (CR). The treatment of relapsed/refractory patients is a challenge and the use of monoclonal therapeutic targets associated with others agents such as immunomodulatory drugs or proteasome inhibitor could be a new way to treat these patients. For example, anti-CD20 monoclonal antibodies (mAb) (rituximab, ofatumumab and obinutuzumab) were evaluated on patients and have improved event-free survival (EFS) and CR in chronic lymphocytic leukemia, another B-cell malignancy (Shah, 2015, Ther Clin Risk Manag,; Vol. 11 : 1113-1122). In MM, anti-CD40, CD38, CD138, CD56, B2-microglobulin, FGFR3 and CS-1 have demonstrated anti-tumor activity in preclinical models and in Phase I/II clinical trials (Donato et al., 2014, Expert Opin Bio Ther , Vol. 14(8): 1-18; van de Donk et al., 2012, Leukemia, Vol. 26 : 199-213). Moreover, the expression of some CD markers is known to be associated with prognosis value such as CD200 (Moreaux et al., 2006, Blood, Vol. 108 : 4194-4197).

Correlation between patient survival and novel markers would allow new prognosis factor identification and a better characterization of the disease including its classification in different stage of evolution. A better knowledge of the MM stage would permit to the clinicians to support patients with personalized treatment.

The invention has for purpose to meet the aforementioned needs.

### SUMMARY OF THE INVENTION

The present invention relates to the use of an expression level value of BLM in a sample obtained from an individual affected with multiple myeloma for predicting responsiveness of the said individual to a treatment of multiple myeloma.

This invention pertains to an *in vitro* method for predicting the responsiveness of an individual affected with multiple myeloma to a treatment of multiple myeloma comprising the steps of :
a) measuring the expression level of BLM in a sample obtained from the said individual,
b) comparing the expression value of BLM measured at step a) with a reference value, and
c) determining the responsiveness of the said individual to a treatment of multiple myeloma from the comparison performed at step b).

In some embodiments of the said *in vitro* prediction method, the expression level of BLM consists of the level of expression of the BLM gene.

In some embodiments of the said *in vitro* prediction method, the expression level of BLM consists of the level of BLM protein expression.

In some embodiments of the said *in vitro* prediction method, a low expression level of BLM is indicative of a good responsiveness of the said individual to a treatment of multiple myeloma with a medicament distinct from a BLM inhibitor.

In some embodiments of the said *in vitro* prediction method, a high expression of BLM is indicative of a good responsiveness of the said individual to a treatment with a BLM inhibitor.

This invention also concerns a method for determining the efficiency of a treatment of multiple myeloma in an individual comprising the steps of;
a) performing an *in vitro* prediction method of the outcome of multiple myeloma on a sample obtained from the said individual before treatment, comprising the steps of:
   a1) measuring the expression level of BLM in a sample obtained from the said individual,
   a2) comparing the expression value of BLM measured at step a1) with a reference value, and
   a3) determining a first prediction of outcome of multiple myeloma in the said individual from the comparison performed at step a2).
b) performing an *in vitro* prediction method of the outcome of multiple myeloma on a sample obtained from the said individual before treatment, comprising the steps of:
   b1) measuring the expression level of BLM in a sample obtained from the said individual,
   b2) comparing the expression value of BLM measured at step b1) with a reference value, and
   b3) determining a second prediction of outcome of multiple myeloma in the said individual from the comparison performed at step b2).
c) determining if the second prediction determined at step a3) means a better outcome than the first prediction determined at step b3).

In some embodiments of the said treatment efficiency determination method, the expression level of BLM consists of the level of expression of the BLM gene.

In some embodiments of the said treatment efficiency determination method, the expression level of BLM consists of the level of BLM protein expression.

This invention also relates to an *in vitro* method for determining the responsiveness of an individual affected with multiple myeloma to a treatment which induces an inhibition of BLM expression, comprising the steps of:
a) measuring the expression level of BLM in a sample obtained from the said individual,
b) comparing the expression value of BLM measured at step a) with a reference value, and
c) determining the responsiveness of the said individual to a treatment which induces an inhibition of BLM expression from the comparison performed at step b).

In some embodiments of the said *in vitro* prediction method, the expression level of BLM consists of the level of expression of the BLM gene.

In some embodiments of the said *in vitro* prediction method, the expression level of BLM consists of the level of BLM protein expression.

The present invention also pertains to a BLM inhibitor for its use for treating an individual affected with multiple myeloma.

It also concerns the use of a BLM inhibitor for preparing a pharmaceutical composition for treating multiple myeloma.

It also pertains to a method for treating multiple myeloma comprising a step of administering a BLM inhibitor to an individual in need thereof.

### DESCRIPTION OF THE FIGURES

**Figure 1****: *BLM* expression in normal and malignant plasma cells.**
   BLM expression in normal BMPCs (N = 5), in MM cells of patients with intramedullary MM (N = 206) and in HMCLs (N = 26). Ordinate : BLM expression, as expressed in Affimetrix signal arbitrary units. Abscissa : from left to right : BMPCs (n=5); MMCs (n=206) and HMCLs (n=26).
**Figure 2****: *BLM* gene expression in the 7 MM molecular subgroups of the TT2-cohort.** PR = proliferation, LB = low bone disease, MS = MMSET, HY = hyperdiploid, CD1 = cyclin D1/D3, CD2 = cyclin D1/D3, MF = MAF. * indicates higher score value compared to all other groups with P<0.05. Ordinate : BLM expression, as expressed in Affimetrix signal arbitrary units. Abscissa : from left to right: PR, LB, MS, HY, CD1, CD2 and MF .
**Figure 3****: BLM expression in MMCs could predict for shorter overall and event free survival in independent cohorts of patients.**
   ***Figure 3A*** ***:*** Patients of the HM cohort (n = 206) were ranked according to increasing BLM expression and a maximum difference in overall survival (OS) was obtained using the Maxstat R function. Upper curve : BLM^{Low}; Lower curve : BLM^{High}. Ordinate : Overall Survival (OS). Abscissa : Days from diagnosis.
   ***Figure 3B*** ***:*** BLM expression is also associated with a shorter event free survival (EFS) in the HM cohort (n = 206). Upper curve : BLM^{Low}; Lower curve : BLM^{High}. Ordinate : Effective Survival (EFS)). Abscissa : Days from diagnosis.
   ***Figure 3C*** ***:*** BLM expression is associated with a poor prognosis (OS) in an independent cohort of 250 patients (LR-TT2 cohort). Upper curve : BLM^{Low}; Lower curve : BLM^{High}. Ordinate : Overall Survival (OS). Abscissa : Days from diagnosis.
   ***Figure 3D*** ***:*** BLM expression is associated with a poor prognosis (EFS) in an independent cohort of 250 patients (LR-TT2 cohort). Upper curve : BLM^{Low}; Lower curve : BLM^{High}. Ordinate : Effective Survival (EFS)). Abscissa : Days from diagnosis.
   ***Figure 3E*** ***:*** BLM is associated with a poor prognosis (OS) in TT3 cohort (n = 158). ). Upper curve : BLM^{Low}; Lower curve : BLM^{High}. Ordinate : Overall Survival (OS). Abscissa : Days from diagnosis.
   ***Figure 3F*** ***:*** BLM is associated with a poor prognosis (OS) in Hovon cohort (n = 282). Upper curve : BLM^{Low}; Lower curve : BLM^{High}. Ordinate : Overall Survival (OS)). Abscissa : Days from diagnosis.
**Figure 4****: ML216 induces a dose-dependent inhibition of human myeloma cell lines (HMCL) cell growth.**
   HMCL were cultured for 4 days in 96-well flat-bottom microtitre plates in RPMI 1640 medium, 10% fetal calf serum, 2 ng/ml interleukin six culture medium (control), and graded concentrations of ML216. At day 4 of culture, the viability was assessed by CellTiter-Glo® Luminescent Cell Viability Assay. The IC50 (concentration responsible for 50% of the maximal inhibitory effect), was determined using GraphPad PRISM software. Data are mean values ± standard deviation (SD) of five experiments determined on sextuplet culture wells.

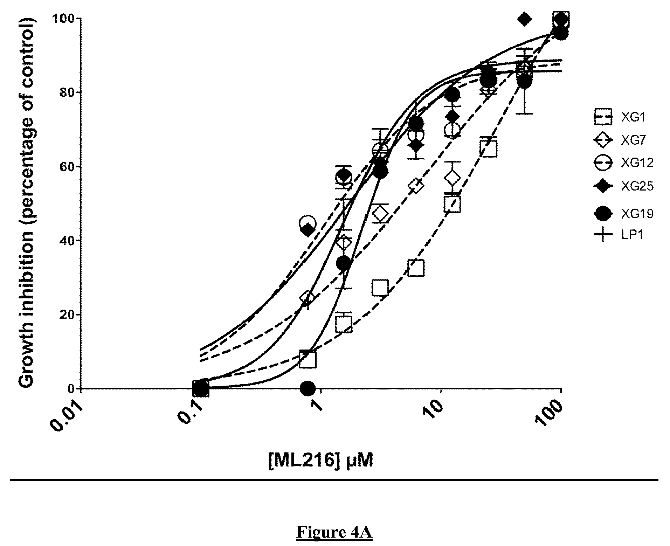

   ***Figure 4A*** ***:*** Growth inhibition of a plurality of MM cell lines in the presence of the ML216 BLM inhibitor. Ordinate : cell growth inhibition, as expressed as percent of control cells without ML216 :Cell lines tested : □ XG1 cells; ◊ XG7 cells; ○ XG12 cells; ◆XG25 cells; ● XG19 cells; + LP1 cells. Abscissa : concentration of ML216, as expressed in µM.

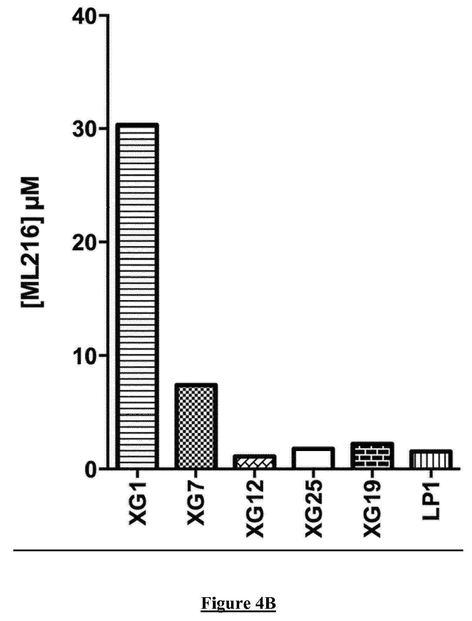

   ***Figure 4B*** ***:*** Growth inhibition of a plurality of MM cell lines in the presence of the ML216 BLM inhibitor. Ordinate : IC50 of ML216 for the indicated MM cell lines. Abscissa : cell lines tested; from left to right: XG1, XG7, XG12, XG25, XG19 and LP1.

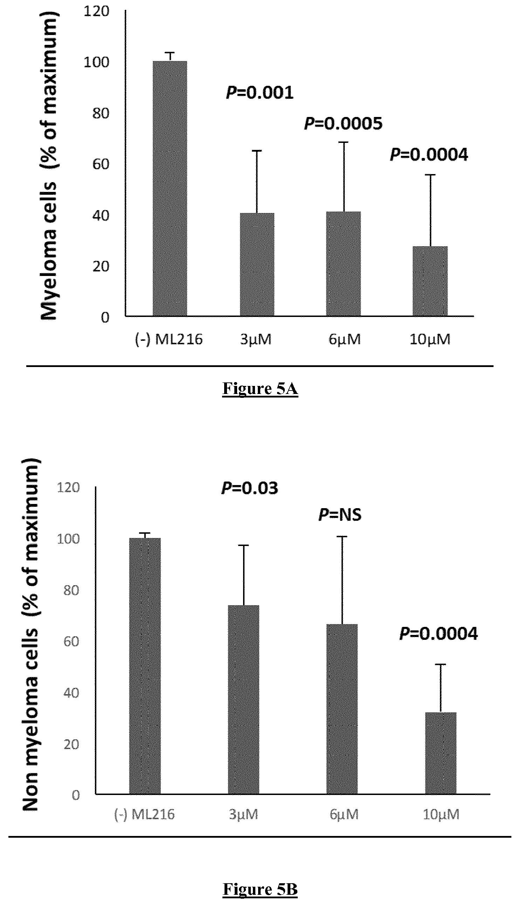

**Figure 5****: ML216 induces a significant toxicity on primary myeloma cells of patients.** Mononuclear cells from 7 patients with MM were cultured for 4 days in the presence of IL-6 (1 ng/mL) with or without graded concentrations of ML216. At day 4 of culture, the viability and total cell counts were assessed and the percentage of CD138 viable plasma and non-myeloma cells was determined by flow cytometry. Results are median values of the numbers of myeloma cells in the culture wells.
   ***Figure 5A*** ***:*** Toxicity of ML216 on primary MM cells. Ordinate : cell viability, as expressed in percent of control cells without ML216. Ordinate : culture conditions; from left to right : (i) without ML216, (ii) ML216 at 3µM, (iii) ML216 at 6µM, (iv) ML216 at 10 µM.
   ***Figure 5B*** ***:*** Toxicity of ML216 on primary non-myeloma cells. Ordinate : cell viability, as expressed in percent of control cells without ML216. Ordinate : culture conditions; from left to right : (i) without ML216, (ii) ML216 at 3µM, (iii) ML216 at 6µM, (iv) ML216 at 10 µM.

### DETAILED DESCRIPTION OF THE INVENTION

The present invention provides for a novel prognosis marker of multiple myeloma outcome in an individual.

The present invention also provides for a novel prognosis marker of the responsiveness of an individual affected with multiple myeloma to a treatment of the said disease.

The inventors have shown that the level of expression of BLM is relevant as a marker of the outcome prognosis of multiple myeloma (multiple myeloma may be also termed "MM" herein).

BLM (also termed Bloom's syndrome helicase) is a RECQ helicase family member. BLM has DNA annealing and unwinding activities. Through its interaction with TOPOIIIα, BLM unwinds the short stretches of naked duplex DNA and processes homologous recombination (HR) intermediates containing a double holiday junction (Larsen et al., 2013, Adv Exp Med Biol, Vol. 767 : 161-184; Chu et al., 2009, Nat Rev Cancer, Vol. 9 (9): 644-654).

The inventors have also shown that the level of expression of BLM is relevant as a marker of the responsiveness of an individual affected with multiple myeloma to a therapeutic treatment of this disease. The inventors' findings show that measuring the level of expression of BLM allows determining or adapting the therapeutic treatment of an individual affected with multiple myeloma. Notably, the inventors' findings show that measuring the level of expression of BLM, especially in MM individuals with a bad outcome prognosis and in MM individuals that do not efficiently respond to conventional treatments of multiple myeloma, allows determining the responsiveness of the said individuals affected with MM to a BLM inhibitor.

Overexpression of BLM in malignant plasma cells of MM patients has been described in the art (Viziteu et al., 2016, Biomarker Research, 4:3 : 1-9). This helicase appears to prefer specific structures including D-loops and Holliday junctions and promotes Holliday junction branch migration(Weinert et al., 2007, Nucleic Acids Res, Vol. 35 (4) : 1367-1376). It may suppress hyper-sister chromatid exchange (SCE) by disruption of D-loop recombination intermediates and also might be involved in the suppression of crossing over during homology-mediated recombination (Sung et al., 2006, Nat Rev Mol Cell Biol, Vol. 7 (10) : 739-750). BLM-mediated crossover suppression may involve synthesis-dependent strand annealing (SDSA. This helicase facilitates telomere replication by resolving G4 structures(Drosopoulos et al., 2015, J Cell Biol, Vol. 210 (2) : 191-208). Defects in BLM are also associated with cancer ((Sung et al., 2006, Nat Rev Mol Cell Biol, Vol. 7 (10): 739-750).

As it will be explained in detail further in the present specification, measuring the level of expression of BLM allows determining a BLM score value, which BLM score value encompasses (i) BLM^{High}, which is a score value associated with a bad MM outcome as well as with a bad responsiveness to a conventional therapeutic treatment of MM and (ii) BLM^{Low}, which is a score value associated with a good MM outcome as well as with a good responsiveness to a conventional therapeutic treatment of MM.

Thus, the inventors have shown that the level of expression of BLM consists of a marker allows a therapeutic orientation of an individual affected with multiple myeloma, including allows the replacement of an ineffective conventional treatment of MM by a treatment comprising an administration of one or more BLM inhibitors. As shown in the examples herein, the relevancy of the expression level of BLM as a marker of the responsiveness to a therapeutic treatment of multiple myeloma has been assessed from data generated from a plurality of independent cohorts of individuals affected with this disease, which include a first cohort of 206 individuals (termed HM cohort in the examples), a second cohort of 245 individuals (termed TT2 cohort in the examples), a third cohort of 158 individuals (termed TT3 cohort in the examples) and a fourth cohort of 282 individuals (termed Hovon cohort in the examples). In these cohorts, the patients underwent frontline high-dose chemotherapy (HDT) and autologous stem cell transplantation.

The therapeutic interest of BLM inhibitor to target myeloma malignant plasma cells from MM that isidentified herein, by using a plurality of patient's cohorts, are confirmed in the examples herein when using (i) human myeloma cell lines and (ii) primary myeloma cells, respectively.

Further, by performing Univariate and Multivariate analysis methods, it is shown in the examples herein that the expression level of BLM consists of a marker of MM, as well as of a marker of the responsiveness to a MM therapeutic treatment, that is independent from all of the other known MM markers tested. These latter results mean that the level of expression of BLM provides further information relating to MM outcome and to the responsiveness of an individual to a therapeutic treatment of MM, that is not provided by any of the other previously known MM markers tested.

Still further, it is shown in the examples that the level of expression of BLM is a marker allowing to discriminate between (i) MM individuals otherwise staged as PR or LB on the one hand, and (ii) MM individuals otherwise staged as MS, HY, CD1, CD2 or MF on the other hand, according to the molecular classification of multiple myeloma disclosed in Zhang et al. (2006, Blood, Vol. 108 (6): 2020-2028).

These further results mean that the level of expression of BLM also consists of a marker allowing to stage individuals affected with multiple myeloma, and notably a marker allowing to predict a good or a bad outcome of multiple myeloma in individuals affected by this disease.

Notably, it is shown in the examples herein that individuals classified as "PR" according to the molecular classification of multiple myeloma of Zhang et al. (2006, *Supra*), which staging category corresponds to bad outcome individuals, possess a score based on the expression level of BLM which is assigned as being BLM^{High}.

Conversely, individuals classified as "MS", "HY", "CD1", "CD2" or "MF", according to the molecular classification of multiple myeloma of Zhang et al. (2006, *Supra*), which staging categories correspond to a better outcome of MM than individuals classified as "PR", possess a score based on the expression level of BLM which is assigned as being BLM^{Low}.

Yet further, the inventors have shown that individuals affected with multiple myeloma, and especially individuals affected with multiple myeloma having a high level of expression of BLM, which include individuals having a BLM^{High} score, which individuals have a poor responsiveness to a conventional therapeutic treatment, may be administered with an effective therapeutic treatment comprising a BLM inhibitor, such as the ML216 BLM inhibitor.

Thus, this invention also provides for a novel prognosis marker of the responsiveness of an individual affected with multiple myeloma to a treatment with a BLM inhibitor.

This invention also provides for the use of a BLM inhibitor for treating an individual affected with multiple myeloma, especially an individual having a high BLM gene expression or a high BLM protein expression, which encompasses individuals having a BLM^{High} score.

ML216 (Available notably as reference #SML0661; Sigma-Aldricht) is a membrane permeable selective inhibitor of Bloom (BLM) helicase. ML216 is selective for BLM over other members of the RecQ family, especially in vivo, and appears to act at the BLM-nucleic acid substrate binding site, inhibiting DNA binding and blocking BLM's helicase activity.

### Methods for predicting outcome of MM or for predicting responsiveness to a therapeutic treatment of MM

The present invention relates to the use of an expression level value of BLM in a sample obtained from an individual affected with multiple myeloma for predicting outcome of multiple myeloma in the said individual.

The present invention also relates to the use of an expression level value of BLM in a sample obtained from an individual affected with multiple myeloma for predicting responsiveness of the said individual to a treatment of multiple myeloma.

BLM gene sequence, BLM mRNA sequence and BLM protein amino acid sequence are described notably in the GenBank database reference n° U39817.1 and in Ellis et al. (1995, Cell, Vol. 84 (3) : 655-666). The BLM gene nucleic acid sequence may also be retrieved (i) from the HGCN database under the accession number 1058, or (ii) from the Entrez_Gene database under the accession number Gene ID: 641. The BLM amino acid sequence may also be retrieved from the UniProt/SwissProt database under the accession number P54132.

The present invention concerns an *in vitro* method for predicting outcome of multiple myeloma in an individual comprising the steps of:
a) measuring the expression level of BLM in a sample obtained from the said individual,
b) comparing the expression level value measured at step a) with a reference value, and
c) determining the predicted outcome of multiple myeloma in the said individual from the comparison performed at step b).

In some embodiments of the *in vitro* prediction method, step c) comprises calculating a prognostic score value, as it will be detailed further in the present specification. The said prognostic score value may also be termed "BLM score value" herein.

The present invention further relates to an *in vitro* method for predicting the responsiveness of an individual affected with multiple myeloma to a treatment of multiple myeloma comprising the steps of:
a) measuring the expression level of BLM in a sample obtained from the said individual,
b) comparing the expression value of BLM measured at step a) with a reference value, and
c) determining the responsiveness of the said individual to a treatment of multiple myeloma from the comparison performed at step b).

In some embodiments of the *in vitro* prediction method, step c) comprises calculating a responsiveness score value.

As used herein, the expression level of BLM encompasses both (i) the level of expression of the BLM gene and (ii) the level of expression of the BLM protein.

Thus, in some embodiments, the expression level of BLM consists of the level of expression of the BLM gene.

Also, in some embodiments, the expression level of BLM consists of the level of expression of the BLM protein.

Within the scope of the invention, "an individual" is intended to mean any mammal, such as cat, dog, preferably a human being, irrespective of its age or gender. In particular, an individual encompassed by the invention is having multiple myeloma. In a preferred embodiment of the invention, an individual refers to any patient (preferably human) afflicted with multiple myeloma. The term "multiple myeloma" refers to multiple myeloma such as revised in the World Health Organisation Classification C90.

An "individual" undergoing a treatment against multiple myeloma encompasses an individual undergoing a therapeutic treatment selected in a group comprising radiotherapy, chemotherapy and/or immunotherapy.

As used herein, a "conventional" treatment of multiple myeloma encompasses any therapeutic treatment of multiple myeloma such as radiotherapy, chemotherapy and/or immunotherapy, except a therapeutic treatment comprising an administration of an inhibitor of BLM expression, either alone or in combination with one or more other active ingredients.

Within the scope of the invention, the term "sample" is intended to mean any biological sample derived from an individual, such as a fluid, including blood, saliva; a tissue; a cell sample, an organ; a biopsy. The term "sample" encompasses a bone marrow-derived sample and a blood-derived sample. In some embodiments, a biological sample refers to multiple myeloma cells, bone marrow or medullary cells.

The sample may be collected according to conventional techniques in the art, and used directly for diagnosis or alternatively stored for subsequent diagnosis. A tumor sample may be fresh, frozen or paraffin- embedded.

In the sense of the invention, "*comprising*" encompasses "*consisting of*" and *"consisting essentially of*".

In some embodiments, a "*reference value*" refers to the expected BLM gene expression level or to the expected BLM protein expression level in the biological sample of an individual that is either (i) a control individual which is not affected with a multiple myeloma or (ii) an individual for whom the occurrence of multiple myeloma is known or detectable. Individuals for whom the occurrence of multiple myeloma is known or detectable encompass (iii) individuals who have not yet received a therapeutic treatment of multiple myeloma, (iv) individuals who have received a therapeutic treatment of multiple myeloma prior to be tested and (v) individuals who are undergoing a therapeutic treatment of multiple myeloma at the time of being tested.

Within the scope of the invention, the expression "reference value" also encompasses a mean value representing a "standard" level of expression of the BLM gene, in conditions wherein the cells express the said BLM gene at a physiological level.

By extension, a reference value can be determined either from a single individual, or from a group of individuals.

In some embodiments, said reference value may represent a mean value measured in a plurality of samples obtained from multiple myeloma-free individual(s). In some embodiments, a plurality of samples encompasses at least 2, at least 3, at least 5, at least 10, at least 15, at least 25, at least 50, at least 100, at least 250, at least 500 samples. In some embodiments, one or more multiple myeloma-free individual(s) encompasses at least 2, at least 3, at least 5, at least 10, at least 15, at least 25, at least 50, at least 100, at least 250, at least 500 multiple myeloma-free individuals.

In some embodiments, said reference value may represent a mean value measured in a plurality of samples obtained from individuals affected with multiple myeloma and who are of a diversity of outcomes.

In some embodiments, the mean reference value may be gathered from a plurality of samples stocked and periodically revaluated.

In certain embodiments, said reference value may represent a mean value measured in a plurality of samples obtained from one or more individual(s) which are not affected with a multiple myeloma.

In some embodiments, a reference value may also represent a value representing a "standard" level of expression of the BLM gene, from a sample obtained from an individual who is affected with multiple myeloma.

In preferred embodiments, a "*reference value*" refers to a median value of expression of the BLM gene or of the BLM protein or a percentage of patients with a high BM expression, or a ratio of between the BLM expression value in malignant myeloma cells and the BLM expression value in non-malignant control cells, which reference value may also be termed "cut-off value".

A BLM gene expression level value that is higher than the said reference value (or "cut-off" value) for gene expression may be assigned a value denoted BLM^{High}. A BLM gene expression level value that is lower than the said reference (or "cut-off') value for gene expression may be assigned a value denoted BLM^{Low}.

Similarly, A BLM protein expression level value that is higher than the said reference value (or "cut-off" value) for protein expression may be assigned a value denoted BLM^{High}. A BLM protein expression level value that is lower than the said reference (or "cut-off") value for protein expression may be assigned a value denoted BLM^{Low}.

Thus, in some embodiments, the level of BLM gene expression or of BLM protein expression may be used as the sole multiple myeloma marker (i) in a method of prognosis of the outcome of multiple myeloma and (ii) in a method of prognosis of the responsiveness of a patient to a therapeutic treatment of multiple myeloma.

In some embodiments, the level of expression of BLM may be used in combination with one or more further multiple myeloma markers (i) in a method of prognosis of the outcome of multiple myeloma and (ii) in a method of prognosis of the responsiveness of a patient to a therapeutic treatment of multiple myeloma.

Within the scope of the invention, the expression "likelihood to efficiently respond to" or "good responsiveness to" is meant to intend that the individual having multiple myeloma may be subjected to stabilization, an alleviating, a curing or a reduction of the progression of the symptoms or the disease itself.

Within the scope of the present invention, the term "symptom" is intended to mean any noticeable response of the body to a multiple myeloma condition. In practice, a symptom encompasses high levels of proteins in the blood, serum, urine, and organs, including but not limited to M-protein and other immunoglobulins (antibodies), albumin, and β2 -microglobulin, as well as hyperviscosity, infections, anemia, hypercalcemia, immunodeficiency, lytic bone lesions, and renal failure, infections, signs and symptoms of light chain amyloidosis.

In some embodiments, individual having a multiple myeloma that efficiently respond to a treatment against multiple myeloma may be characterized by a significant reduction of the size of the tumor, a reduction of the invasive properties of the tumor cells, a reduction of the migration properties of the tumor cells, a reduction of the mean cancerous cells counts, a variation of known multiple myeloma biomarkers.

Each of these parameters may be evaluated according to known methods routinely used in the art.

Within the scope of the invention, the expression "measuring the level of expression of BLM" is intended to mean quantitatively or semi-quantitatively measuring either the level of the BLM protein expression or the level of the BLM gene expression in the cells comprised within the sample.

### Measuring BLM protein expression level

In practice, the measurement of the expression level of the BLM protein may be performed accordingly to any known method in the art.

In some embodiments, said method may comprise a step of contacting the sample with a compound capable of selectively binding to the BLM protein, said compound being subsequently detected. For example, a compound capable of selectively binding to the BLM protein may be an antibody, such as, for example, a monoclonal antibody, or an aptamer.

In some embodiments, the level of BLM protein expression may be measured by using standard immunodiagnostic techniques, including immunoassays such as competition, direct reaction, or sandwich type assays. Such assays include, but are not limited to, agglutination tests; enzyme-labelled and mediated immunoassays, such as ELISAs; biotin/avidin type assays; radioimmunoassays; immunoelectrophoresis; immunoprecipitation; flow cytometry, immunohistochemical staining. In some embodiments, a compound capable of selectively binding to the BLM protein may be immobilized onto a suitable solid support, prior to its use.

In some preferred embodiments, the level of BLM protein expression is measured by a flow cytometry method.

In some preferred embodiments, the level of BLM protein expression is measured by mass spectrometry.

In embodiments wherein the expression level of BLM consists of the expression level of the BLM protein, any known flow cytometry method may be used, especially a flow cytometry method using an antibody specifically directed against the BLM protein.

In some other embodiments, the level of the BLM protein expression may be measured by immunohistochemical staining, preferably by using a monoclonal antibody directed against the BLM protein.

In some embodiments, monoclonal antibodies directed against the BLM protein may be prepared accordingly to any method known from the state in the art, e.g. as described in Harlow and Lane ("Antibodies: A Laboratory Manual", 2nd Ed; Cold Spring Harbor Laboratory Press, Cold Spring Harbor, NY, 1988).

In practice, in order to perform immunohistochemistry, tissue sections may be obtained from a individual and fixed onto a glass slide by any suitable fixing agent, such as e.g. alcohol, acetone, and paraformaldehyde, to which is reacted an antibody. Conventional methods for immunohistochemistry are described in Harlow and Lane ("Antibodies A Laboratory Manual", Cold Spring Harbor Press, Cold Spring Harbor, New York, 1988), Ausubel et al. ("Current Protocols In Molecular Biology", John Wiley and Sons, NY, 1987) or Brauer et al. (FASEB J, 15, 2689-2701; 2001).

In some embodiments, commercially available monoclonal antibody directed against the BLM protein may be suitable, such as :
- anti-BLM antibody #2742 marketed by Cell Signaling Technology (Ozyme, Saint Quentin en Yvelines, France),
- anti-BLM antibody #ab476 marketed by Abcam (Paris, France), and
- anti-BLM antibody #ab2179 marketed by Abcam (Paris, France),

In some embodiments, the measure of the level of expression of the BLM protein may be dependent of the intensity of the labelling obtained from the anti-BLM protein antibody used and/or the number of cells expressing a noticeable amount of the said BLM protein.

### Measuring BLM gene expression level

In practice, the measurement of the level of the BLM gene expression may be performed according to any known method in the art.

Determination of the expression level of the genes can be performed by a variety of techniques. Generally, the expression level as determined is a relative expression level. More preferably, the determination comprises contacting the biological sample with selective reagents such as probes, primers or ligands, and thereby detecting the presence, or measuring the amount, of polypeptide or nucleic acids of interest originally in the biological sample. Contacting may be performed in any suitable device, such as a plate, microtiter dish, test tube, well, glass, column, and so forth. In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the biological sample. In a preferred embodiment, the expression level may be determined by determining the quantity of mRNA.

In some embodiments, the measurement of the level of the BLM gene expression is performed by the measurement of the quantity of mRNA, notably by a method routinely used in the art. In practice, the total nucleic acids fraction contained in the sample may be obtained according to standard methods, such as using lytic enzymes or chemical solutions or extracted by nucleic-acid-binding resins following the manufacturer's instructions. The extracted mRNA may be subsequently detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

In some embodiments, other methods of amplification including ligase chain reaction (LCR), transcription-mediated amplification (TMA), strand displacement amplification (SDA) and nucleic acid sequence based amplification (NASBA) may be used.

In practice, probes for hybridization and/or amplification may be designed and obtained by any known method in the art.

In some other embodiments, the measurement of the level of BLM gene expression is performed by the DNA chip analysis technology (Hoheisel, Nature Reviews, Genetics, 2006, 7:200-210). Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210)

In some embodiments, the level of expression of the BLM gene may be determined by using a micro-chip selected in a group comprising the micro-chips referenced as Affymetrix Human Genome U133 Plus 2.0 Arrays, which are marketed by Affymetrix (Santa Clara, CA, USA). After hybridization, the micro-chip arrays are scanned following guidelines from the manufacturer. These Affymetrix Human Genome U133 Plus 2.0 micro-chip contain ∼54,000 probesets, representing ∼47,000 transcripts..

Methods for determining the quantity of mRNA are well known in the art. For example the nucleic acid contained in the biological sample is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of amplification include ligase chain reaction (LCR), transcription-mediated amplification (TMA), strand displacement amplification (SDA) and nucleic acid sequence based amplification (NASBA).

Methods for determining the quantity of mRNA by RNA sequencing may also be used.

In preferred embodiments wherein the level of the BLM gene expression is performed by the measurement of the quantity of mRNA, any known RNA level measuring method may be used, and especially it may be used the Prime Flow@ method of measuring the RNA level by flow cytometry.

Thus, BLM gene expression may be combined with flow cytometry approach using PrimeFlow® technology representing a powerful tool for risk stratification and outcome prediction in MM. The PrimeFlow® technology expands the typical flow cytometry application by allowing simultaneous detection of RNA transcripts and proteins in single cells. The assay, which is notably called the PrimeFlow™ RNA Assay, facilitates analysis of transcript and protein expression correlations as the cell changes over time or in response to stimulus. The assay employs a fluorescent in situ hybridization (FISH) technique for simultaneous detection of up to three RNA transcripts in a single cell using a standard flow cytometer. RNA detection may be teamed together with intracellular and cell surface antibody staining to elevate the understanding of single cell dynamics to a new dimension.

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from a biological sample and subjecting the RNAs to amplification and hybridization to specific probes, more particularly by means of a quantitative or semi-quantitative RT-PCR.

In this context, the invention further provides a DNA chip comprising a solid support which carries nucleic acids that are specific to the BLM gene expression product(s).

### Score values

In some embodiments, the measured expression value of the BLM gene or of the BLM ptotein may be classified as (i) a "high" expression value or (ii) a "low" expression value, respectively.

In some embodiments, regarding attribution of a score value, a 2 grades scoring may be attributed according to the measured level of the BLM gene expression level or of the BLM protein expression level, namely:
- a good outcome prognosis score when a low expression level of the BLM gene or of the BLM protein is measured,
- a bad outcome prognosis score when a high expression level of the BLM gene or of the BLM protein is measured.

As used above for attributing a two grades scoring for outcome prediction,
- a "low" expression level of the BLM gene is determined when the BLM expression level value that is measured is lower than a selected outcome reference value, and
- a "high" expression level of the BLM gene is determined when the BLM expression level value that is measured is higher than a selected outcome reference value.

Within the scope of the invention, the expression "good outcome" encompasses any amelioration of the medical condition, including stabilization, an alleviating, reduction of the progression of the symptoms or the disease itself, an extended survival time period, an extended relapse-free time period or a total remission.

Within the scope of the invention, the expression "good outcome" also encompasses a benefit in term of overall survival prognosis. In some embodiments, an overall survival prognosis benefit may account for a significant gain of life expectancy.

As also shown in the examples herein, a prognosis score corresponding to a good outcome may mean that the tested individual is at low risk and has a median event-free time period (EFS) and overall survival (OS) substantially longer than the median event-free time period (EFS) and overall survival (OS) of a tested individual having a prognosis score corresponding to a bad outcome.

In some other embodiments, regarding attribution of a score value, a 2 grades scoring may be attributed accordingly to the measured level of the BLM gene expression level or of the BLM protein expression level, namely:
- a good responsiveness score to a conventional therapeutic treatment of multiple myeloma when a low expression level of the BLM gene or of the BLM protein is measured,
- a bad responsiveness score to a conventional therapeutic treatment of multiple myeloma when a high expression level of the BLM gene or of the BLM protein is measured.

As used above for attributing a two grades scoring for treatment responsiveness prediction,
- a "low" expression level of the BLM gene is determined when the BLM expression level value that is measured is lower than a selected responsiveness reference value, and
- a "high" expression level of the BLM gene is determined when the BLM expression level value that is measured is higher than a selected responsiveness reference value

It shall be understood that the same reference value may be used both as (i) a reference value for a two grades scoring for MM outcome prediction and as (ii) a reference value for a two grades scoring of responsiveness to a treatment of MM.

As shown in the examples herein, the score which may be calculated from the BLM expression level may be indicative of the predicted likelihood of said individual having a multiple myeloma to efficiently respond to a conventional therapeutic treatment against multiple myeloma, i.e. a therapeutic treatment of multiple myeloma that does not comprise an administration of a BLM expression inhibitor, either alone or in combination with one or more other active ingredients.

In some embodiments, an individual having a multiple myeloma responding efficiently to a conventional therapeutic treatment may be characterized by a better efficiency of the active ingredient(s) used according to the said conventional therapeutic treatment against multiple myeloma. Conventional therapeutic treatment of multiple myeloma encompasses chemotherapy, exposure to radiation, surgery, immunotherapy, stem cell transplant and plasmapheresis provided that the said therapeutic treatment does not comprise an administration of a BLM expression inhibitor, either alone or in combination with one or more other active ingredients.

Many different types of drugs are used to treat multiple myeloma, which encompass Melphalan, Vincristine (Oncovin), Proteasome inhibitors, Cyclophosphamide (Cytoxan), Etoposide (VP-16), Doxorubicin (Adriamycin), Liposomal doxorubicin (Doxil) and Bendamustine (Treanda). Combinations of these drugs are more effective than any single drug. Often these drugs are combined with other types of drugs like corticosteroids, bisphosphonates, immunomodulating agents (drugs that will change the patient's immune response), antibodies or epigenetic agents.

Radiation therapy uses focused high-energy x-rays or particles that penetrate the tissues of the body to reach and destroy cancer cells. Radiation may be used to treat areas of bone damaged by myeloma that have not responded to chemotherapy and are causing pain. It's also the most common treatment for solitary plasmacytomas. The type of radiation therapy most often used to treat multiple myeloma or solitary plasmacytoma is called *external beam radiation therapy.*

Although surgery is sometimes used to remove single plasmacytomas, it's rarely used to treat multiple myeloma. When spinal cord compression causes paralysis, severe muscle weakness, or numbness, emergency surgery may be needed.

In a stem cell transplant, the patient gets high-dose chemotherapy (sometimes with radiation to the whole body) to kill the cells in the bone marrow (including normal hematopoietic cells and myeloma cells). Then the patient receives new, healthy hematopoietic stem cells. When stem cell transplants were first developed, the new stem cells came from bone marrow, and so this was known as a *bone marrow transplants.* Now, stem cells are more often gathered from the blood (a peripheral blood stem cell transplant).

Stem cell transplant is commonly used to treat multiple myeloma. Before the transplant, drug treatment is used to reduce the number of myeloma cells in the patient's body. Stem cell transplants (SCT) encompass autologous stem cell transplants and allogeneic stem cell transplant.

In some embodiments, the outcome of multiple myeloma may be determined before a medical treatment intended to cure or reduce the disease. Said outcome may be determined within few days or few months prior initiating a therapeutic treatment intended to cure or reduce multiple myeloma.

In some embodiments, the outcome may be determined during the course of a medical treatment intended to cure or reduce multiple myeloma.

In some other embodiments, the outcome may be determined after a medical treatment intended to cure or reduce multiple myeloma. Said outcome may be determined within few days or few months after the end of the said therapeutic treatment.

In some embodiments, any determination of the outcome of an individual having a multiple myeloma, by measuring the BLM expression level as disclosed herein, may be undertaken in order to adjust the medical treatment, which encompasses maintaining or stopping the treatment, increasing or decreasing the doses of the pharmaceutically active agent(s) to be administered, increasing or decreasing the time interval between two administrations of the pharmaceutically active agent(s).

In a one aspect, the invention relates to a use for predicting a likelihood of an individual having a multiple myeloma to efficiently respond to a conventional therapeutic treatment against multiple myeloma.

As shown in the examples section below, the level of BLM expression is significantly correlated with the efficacy of a conventional therapeutic treatment of multiple myeloma.

In some embodiments, the level of expression of BLM for use as a biomarker may be combined to one or more other known biomarker(s) for predicting an outcome for an individual having a multiple myeloma or for predicting a likelihood of an individual having a multiple myeloma to efficiently respond to a conventional therapeutic treatment against multiple myeloma.

In some embodiments, other biomarkers may be selected in a group comprising UAMS 70-gene (Blood, 2006, Vol. 109 : 2276-2284), IFM signature (J Clin Oncol, 2008, Vol. 26 : 4798-4805), Centrosome Index (Blood, 2008, Vol. 111 : 1603-1609), HZD cell death signature (Clin Cancer Res, 2010, Vol. 16 : 1856-1864), IL6-HMCL signature (Haematologica, 2011, Vol. 96 ; 574-582), RS score (Bioinformatics, 2013, Vol. 29 ; 1149-1157), Proliferation Index (Haematologica, 2011, Vol. 96 : 87-95), EMC 92 gene signature (Leukemia, 2012, Vol. 26 : 2406-2413) and Chromosome Instability genomic event count (CINGEC) signature (PLoS One, 2013, 8 : e66361).

In a one aspect, the invention relates to a use of a level of expression of the BLM gene of the level of expression of the BLM protein as a biomarker for determining a severity of multiple myeloma in an individual.

As disclosed in the examples herein, the BLM expression level value, or the score value calculated from the said BLM expression level value, allows a MM staging of the tested individual. As disclosed in the examples, a high BLM expression value, or alternatively a high BLM expression score value, is associated with a poor outcome in term of EFS and OS in several independent cohorts of patients. Furthermore, it is indicative of a staging class of bad outcome, such as a PR staging class, according to the classification by Zhang et al. (2006, Blood, Vol. 108 (6) : 2020-2028). As it is also disclosed in the examples, a low BLM expression value, or alternatively a low BLM expression score value, is indicative of a staging class of a good outcome, such as an outcome associated with MS, HY, CD1, CD2 or MF staging classes, , according to the classification by Zhang et al. (2006, Blood, Vol. 108 (6) : 2020-2028).

In some embodiments of the *in vitro* prediction methods described herein, the measured value of the BLM expression level is used by taking into account Expression Level Reference (ELRI)(calculated using Maxstat algorithm) for BLM. These parameters are listed in Table 1 hereunder. The ELRI reference level value for BLM is of 1450. In other embodiments, the reference level may be a percentage cut-off value of patients with a high BLM expression or a ratio value between the BLM expression level value in malignant plasma cells versus the BLM expression level value in non-malignant control cells.

### Embodiments of the prediction methods

As already specified elsewhere in the present specification, the level of BLM expression may be used to define a score value, wherein :
- the said score value is indicative of the prognosis of, or of the severity of, multiple myeloma in a tested individual, or
- the said score value is indicative of the responsiveness of the tested individual to a therapeutic treatment of multiple myeloma.

As it is also shown herein, a relevant score value based on the BLM expression level may be determined according to various methods, which encompass calculating the reference value of BLM.

In some embodiments, the BLM expression score value consists of a numerical value according to which a good outcome status or a bad outcome status of the tested individual may be determined.

In some other embodiments, the BLM expression score value is directly the status of the tested patient, the said status being selected in the group consisting of (i) a good outcome status and (ii) a bad outcome status.

In some further embodiments, the BLM expression score value is a numerical value according to which a good responsiveness or a bad responsiveness to a conventional therapeutic treatment of multiple myeloma may be determined.

As it is detailed further in the present specification, a BLM gene expression value or a BLM protein expression value measured that is measured may be labeled "High" if the measured expression value is higher than a reference value and (ii) may be labeled "Low" if the measured expression value is lower than a reference value. In some embodiments, the said reference value is a "cut-off" reference value, such as a score value which is termed (ELRi), the calculation of which is detailed further in the present specification.

Predetermined reference values ELRi used for comparison may consist of "cut-off' values.

For example; each reference ("cut-off") value ELRi for BLM may be determined by carrying out a method comprising the steps of:
a) providing a collection of samples from patients suffering of multiple myeloma;
b) determining the expression level of the relevant gene for each sample contained in the collection provided at step a);
c) ranking the samples according to said expression level
d) classifying said samples in pairs of subsets of increasing, respectively decreasing, number of members ranked according to their expression level,
e) providing, for each sample provided at step a), information relating to the actual clinical outcome for the corresponding cancer patient (i.e. the duration of the event-free survival (EFS) or the overall survival (OS) or both);
f) for each pair of subsets of tumour tissue samples, obtaining a Kaplan Meier percentage of survival curve;
g) for each pair of subsets of tumour tissue samples calculating the statistical significance (p value) between both subsets
h) selecting as reference value ELR for the expression level, the value of expression level for which the p value is the smallest.

In some other embodiments, the reference value is determined as a percentage value; e.g. the percentage of MM patients having a high BLM expression.

In some further embodiments, the reference value is determined as a ratio value of (i) the BLM expression value in malignant plasma cells against (ii) the BLM expression value in non-malignant cells.

For example, the expression level of the BLM gene may be assessed for 100 samples of 100 patients. The 100 samples are ranked according to the expression level of the BLM gene. Sample 1 has the highest expression level and sample 100 has the lowest expression level. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding cancer patient, Kaplan Meier curves are prepared for each of the 99 groups of two subsets. Also for each of the 99 groups, the p value between both subsets was calculated. The reference value ELRi is then selected such as the discrimination based on the criterion of the minimum p value is the strongest. In other terms, the expression level corresponding to the boundary between both subsets for which the p value is minimum is considered as the reference value. It should be noted that according to the experiments made by the inventors, the reference value ELRi is not necessarily the median value of expression levels.

According to these embodiments, a measured expression value that is higher than the reference value may be expressed as BLM^{High}.

According to these embodiments, a measured expression value that is lower than the reference value may be expressed as BLM^{Low}.

Thus, in some embodiments of the *in vitro* prediction method described herein, a good outcome status may be determined at the final step of this method for the marker status denoted BLM^{Low}.

Also, in some embodiments of the *in vitro* prediction method described herein, a bad outcome status may be determined at the final step of this method for the following marker status denoted BLM^{High}.

The present invention also relates to an *in vitro* method for predicting outcome of multiple myeloma in an individual comprising the steps of:
(a) determining the expression level (ELi) of BLM in a sample obtained from the said individual,
(b) comparing the expression level (ELi) determined at step (a) with a predetermined reference level (ELRi)
(c) calculating a BLM expression value, and :
(d) determining the predicted outcome of multiple myeloma in the said individual from the comparison performed at step (c).

In some embodiments of the *in vitro* prediction method described herein, a good outcome status may be determined at the final step of this method for the marker status denoted BLM^{Low}.

In some embodiments of the *in vitro* prediction method described herein, a bad outcome status may be determined at the final step of this method for the following marker status denoted BLM^{High}.

### Methods for determining the efficiency of a conventional therapeutic treatment of multiple myeloma

The present invention also relates to an *in vitro* method for determining the efficiency of a therapeutic treatment of multiple myeloma in an individual comprising the steps of;
a) performing the *in vitro* prediction method described herein on a sample obtained from the said individual before receiving the said therapeutic treatment, so as to determine a first prediction of outcome of multiple myeloma in the said individual,
b) performing the *in vitro* prediction method described herein on a sample obtained from the said individual after having received the said therapeutic treatment, so as to determine a second prediction of outcome of multiple myeloma in the said individual,
c) determining if the second prediction means a better outcome than the first prediction.

The present invention further concerns an *in vitro* method for determining the efficiency of a conventional therapeutic treatment of multiple myeloma in an individual comprising the steps of;
a) performing the *in vitro* prediction method described herein on a sample obtained from the said individual before receiving the said conventional therapeutic treatment, so as to determine a first prediction of outcome of multiple myeloma in the said individual,
b) performing the *in vitro* prediction method described herein on a sample obtained from the said individual after having received the said conventional therapeutic treatment, so as to determine a second prediction of outcome of multiple myeloma in the said individual,
c) determining if the second prediction means a better outcome than the first prediction.

This invention further pertains to a method for determining the efficiency of a conventional therapeutic treatment of multiple myeloma in an individual comprising the steps of;
a) performing an *in vitro* prediction method of the outcome of multiple myeloma on a sample obtained from the said individual before receiving the said conventional therapeutic treatment, comprising the steps of :
   a1) measuring the expression level of BLM in a sample obtained from the said individual,
   a2) comparing the expression value of BLM measured at step a1) with a reference value, and
   a3) determining a first prediction of outcome of multiple myeloma in the said individual from the comparison performed at step a2).
b) performing an *in vitro* prediction method of the outcome of multiple myeloma on a sample obtained from the said individual after having received the said conventional therapeutic treatment, comprising the steps of :
   b1) measuring the expression level of BLM in a sample obtained from the said individual,
   b2) comparing the expression value of BLM measured at step b1) with a reference value, and
   b3) determining a second prediction of outcome of multiple myeloma in the said individual from the comparison performed at step b2).
c) determining if the second prediction determined at step a3) means a better outcome than the first prediction determined at step b3).

The present invention also pertains to an *in vitro* method for determining the efficiency of a conventional therapeutic treatment of multiple myeloma in an individual comprising the steps of;
a) performing an *in vitro* prediction method of the outcome of multiple myeloma on a sample obtained from the said individual before receiving the said conventional therapeutic treatment, comprising the steps of :
   (a1) determining the expression level (ELi) of BLM in a sample obtained from the said individual,
   (a2) comparing the expression level (ELi) determined at step (a) with a predetermined reference level (ELRi)
   (a3) calculating BLM expression score value, and
   (a4) determining a first prediction of outcome of multiple myeloma in the said individual from the comparison performed at step (a3).
b) performing an *in vitro* prediction method of the outcome of multiple myeloma on a sample obtained from the said individual after having received the said conventional therapeutic treatment, comprising the steps of :
   (b1) determining the expression level (ELi) of BLM in a sample obtained from the said individual,
   (b2) comparing the expression level (ELi) determined at step (a) with a predetermined reference level (ELRi)
   (b3) calculating BLM expression score value, and
   (b4) determining a second prediction of outcome of multiple myeloma in the said individual from the comparison performed at step (a3).
c) determining if the second prediction determined at step a4) means a better outcome than the first prediction determined at step b4).

In some embodiments of the *in vitro* prediction method described herein, a good therapeutic efficiency may be determined at the final step of this method when the second prediction of outcome is better than the first prediction of outcome.

In some embodiments of the *in vitro* prediction method described herein, a bad therapeutic efficiency may be determined at the final step of this method when the second prediction of outcome is similar, identical or worse than the first prediction of outcome.

### Methods for determining responsiveness to a therapeutic treatment of multiple myeloma

### inducing an inhibition of BLM

This invention also concerns an *in vitro* method for determining the responsiveness of an individual affected with multiple myeloma to a treatment which induces an inhibition of BLM, comprising the steps of :
a) measuring the expression level of BLM in a sample obtained from the said individual,
b) comparing the expression value of BLM measured at step a) with a reference value, and
c) determining the responsiveness of the said individual to a treatment which induces an inhibition of BLM expression from the comparison performed at step b).

In some embodiments, a good responsiveness of the said individual to a treatment which inhibits BLM may be determined at the final step of this method for the marker status denoted BLM^{High}.

In some embodiments, a bad responsiveness of the said individual to a treatment which inhibits BLM may be determined at the final step of this method for the marker status denoted BLM^{Low}.

In some embodiments of the methods above, the expression level of BLM consists of the level of expression of the BLM gene.

In some embodiments of the methods above, the expression level of BLM consists of the level of BLM protein expression.

In some embodiments of the methods above, the said sample is selected in a group comprising a bone marrow-derived sample and a blood-derived sample.

In some embodiments, a therapeutic treatment of MM which induces an inhibition of BLM expression includes a therapeutic treatment by administration of (i) one or more inhibitors of BLM helicase activity, (ii) one or more inhibitors of the BLM gene expression and of (ii) one or more inhibitors of the BLM protein expression.

Inhibitors of BLM helicase activity encompass ML216, which is a small molecule inhibitor that is notably disclosed by Rosenthal et al. (2013, Probe Reports from the NIH Molecular Libraries Program, Bethesda (MD, USA) : National Center for Biotechnology Information) and by Nguyen et al. (2013, Chem Biol., Vol. 20(1) : 55-62). ML216 BLM inhibitor is notably available from Calbiochem (Fontenay sous bois, France) under the reference #506384.

Inhibitors of BLM helicase encompasses antibodies directed against BLM, preferably monoclonal antibodies directed against BLM.

Inhibitors of BLM gene expression encompasses antisense nucleotides, that may be easily prepared to methods well known from the one skilled in the art, since the BLM gene sequence is publicly known.

### Kits

The invention also relates to a kit for performing the methods as above described, wherein said kit comprises means for measuring the expression level of the BLM gene or of the BLM protein. Typically the kit may include a primer, a set of primers, a probe, a set of probes, a method of sequencing, as a set of antibodies above described. In a particular embodiment, the probe or set of probes are labelled as above described. The kit may also contain other suitably packaged reagents and materials needed for the particular detection protocol, including solid-phase matrices, if applicable, and standards.

In some embodiments, the BLM expression score value may be generated by a computer program.

### Medical uses

The methods described herein allows to define a subgroup of patients who will be responsive ("responder") or not responsive ("non responder") to a conventional treatment of multiple myeloma, e.g. to a treatment with a therapeutic compound, which encompasses a proteasome inhibitor such as bortezomib, mephalan, or with a combination therapy such as with a combination of dexamethasone and an immunomodulatory drug, a combination of bortezomib, mephalan and prednisone, a combination of mephalan, prednisone and thalidomide, as illustrative embodiments, provided that the said conventional treatment does not comprise an administration of a BLM inhibitor, either alone or in combination with one or more other active ingredients.

A further object of the invention relates to methods for the treatment of multiple myeloma in a patient in need thereof.

In the context of the invention, the term "treating" or "treatment", as used herein, means reversing, alleviating, inhibiting the progress of, or preventing the disorder or condition to which such term applies, or one or more symptoms of such disorder or condition.

In some embodiments, the method comprises the following steps
a) testing whether the patient will respond or not to a conventional therapeutic treatment by performing the *in vitro* prediction method described herein,
b) administering the said conventional therapeutic treatment, if said patient has BLM expression score value related to high risk (i.e. the patient will respond to the said therapeutic compound).

In some embodiments, the said conventional therapeutic treatment is a proteasome inhibitor such as bortezomib.

A further object of the invention relates to a therapeutic compound for its use in the conventional therapeutic treatment of multiple myeloma in a patient in need thereof, wherein the patient was being classified as responder to the said therapeutic compound by the *in vitro* prediction method disclosed herein.

The present invention also relates to a method for treating an individual affected with multiple myeloma comprising the steps of:
a) performing the *in vitro* method for predicting responsiveness of the said individual to a treatment of multiple myeloma as described herein, and
b) administering a BLM inhibitor to the said individual if a good responsiveness to a treatment with a BLM inhibitor has been determined at step a).

This invention also pertains to a method for treating an individual affected with multiple myeloma comprising a step of administering to the said individual a BLM inhibitor.

This invention also relates to a BLM inhibitor for its use for treating an individual affected with multiple myeloma. In preferred embodiments, the said individual is selected as being a responder to a treatment with a BLM inhibitor by performing a method of determining responsiveness to a BLM inhibitor as described herein.

The invention will be further illustrated, without being limited to, by the following figures and examples.

### EXAMPLES

### A. MATERIAL AND METHODS

### A.1. Human myeloma cell lines

Human myeloma cell lines (HMCLs, n = 6) XG-1, XG-7, XG-12, XG-25, XG-19 and LP1 were obtained as previously described (25) or purchased from Deutsche Sammlung von Mikroorganismen und Zellkulturen GmbH (Braunschweig, Germany) and American Type Culture Collection (Rockville, MD, USA). Microarray data are deposited in the ArrayExpress public database (accession numbers E-TABM-937 and E-TABM-1088). HMCL were cultured with graded Chetomin (Sigma, St Louis, MO, USA) concentrations. HMCL cell growth was quantified with a Cell Titer Glo Luminescent Assay (Promega, Madison, WI, USA) and the 50% inhibitory concentration (IC₅₀) was determined using GraphPad Prism software (http://www.graphpad.com/scientific-software/prism/).

### A.2. Patient samples and gene expression data

Gene expression profiling (GEP) of MMCs were obtained from two independent large patients' cohorts: the Heidelberg-Montpellier (HM, N=206) cohort (ArrayExpress public database under accession number E-MTAB-362) (Hose et al., 2011, Haematologica, Vol. 96 (1) : 87-95; Moreaux et al. Haematologica 2011 Apr;96(4):574-82.) and the University of Arkansas for Medical Sciences (UAMS, Little Rock, USA, cohort treated with total therapy 2, N=345) (GEO, http:// www.ncbi.nlm.nih.gov/geo/, accession number GSE2658).

Bone marrow of patients presenting with previously untreated MM (N = 7) at the university hospital of Montpellier was obtained after patients' written informed consent in accordance with the Declaration of Helsinki and agreement of the Montpellier University Hospital Centre for Biological Resources (DC-2008-417). Primary myeloma cells of patients were cultured with or without graded concentrations of ML216 and MMC cytotoxicity evaluated using anti-CD138-phycoerythrin monoclonal antibody (Immunotech, Marseille, France) as described (Moreaux et al. Molecular Cancer Therapeutics 2012).

### A.3. Gene expression profiling and statistical analyses

Prognostic significance was defined using Maxstat R function and Benjamini Hochberg multiple testing correction. The statistical significance of differences in overall survival (OS) and EFS between groups of patients was calculated by the log-rank test. Multivariate analysis was performed using the Cox proportional hazards model. Survival curves were plotted using the Kaplan-Meier method. All these analyses have been done with R.2.10.1 and Bioconductor version 2.5.

### A.3. Gene Set Enrichment Analysis (GSEA)

The gene expression levels from BLM high risk group versus low risk MM patients were compared and the genes which had significant different expression for GSEA were picked up. Gene set enrichment analysis was carried out by computing overlaps with canonical pathways and gene ontology gene sets obtained from the Broad Institute (Cambridge, MA, USA).

### A.4. Cell growth inhibition

HMCL were cultured for 4 days in 96-well flat-bottom microtitre plates in RPMI 1640 medium, 10% fetal calf serum, 2 ng/ml interleukin six culture medium (control), and graded concentrations of ML216. At day 4 of culture, the viability was assessed by CellTiter-Glo® Luminescent Cell Viability Assay. The IC50 (concentration responsible for 50% of the maximal inhibitory effect), was determined using GraphPad PRISM software. Data are mean values ± standard deviation (SD) of five experiments determined on sextuplet culture wells.

### A.5. Cytotoxicity

Mononuclear cells from 7 patients with MM were cultured for 4 days in the presence of IL-6 (1 ng/mL) with or without graded concentrations of ML216. At day 4 of culture, the viability and total cell counts were assessed and the percentage of CD138 viable plasma and non-myeloma cells was determined by flow cytometry. Results are median values of the numbers of myeloma cells in the culture wells.

### B. RESULTS

### Example 1 : BLM expression as a prognosis marker of Mutiple Myeloma (MM) and of the responsiveness of an individual affected with MM to a therapeutic treatment of MM.

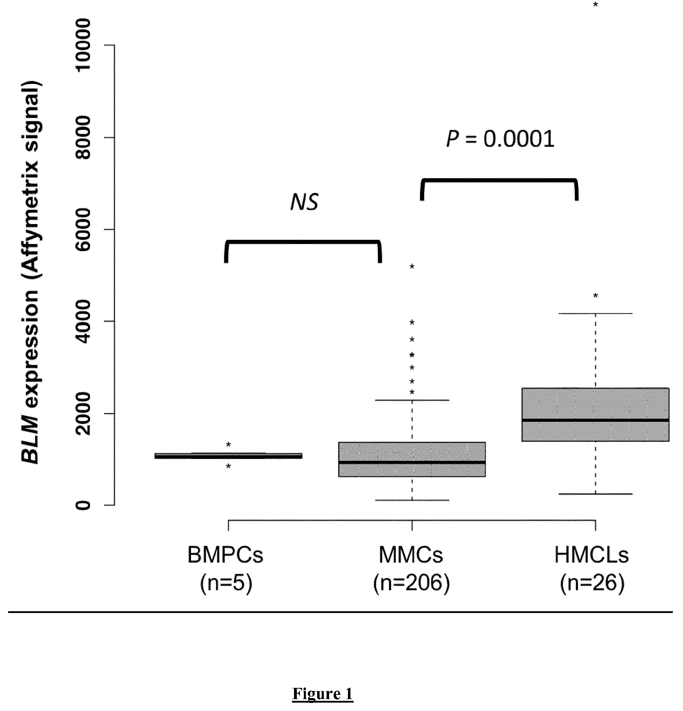

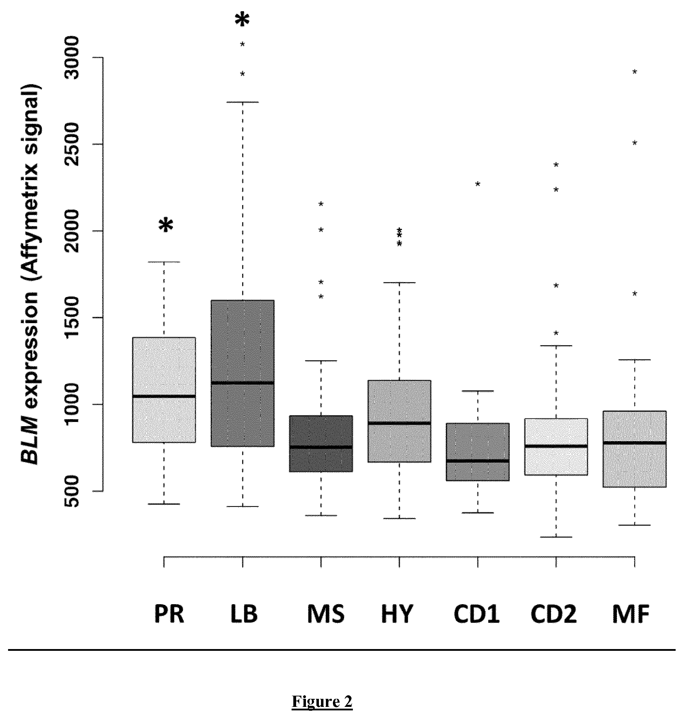

*BLM* gene was not significantly differentially expressed between MMCs of patients (median 935, range 113 - 5206) compared to normal BMPCs (median = 841; range: 523 - *1519)(P* < .001). However, abnormal spiked expression was identified in several patients (*P* < .001). The expression was significantly higher in HMCLs (median 1848, range 246 - 10901) compared to primary MMCs or BMPCs (*P* < .001) (Figure 1). Primary MMCs of previously-untreated patients can be classified into 7 molecular groups associated with different patients' survival(16). *BLM* expression was significantly higher in the poor prognosis proliferation group (*P* < 0.05) and in the LB (Low bone disease) group (*P* < 0.01) (Figure 2).

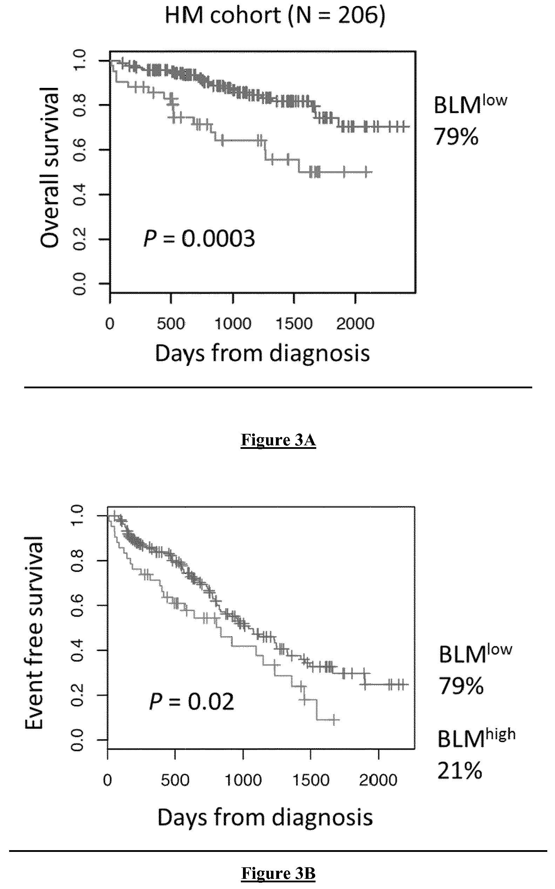

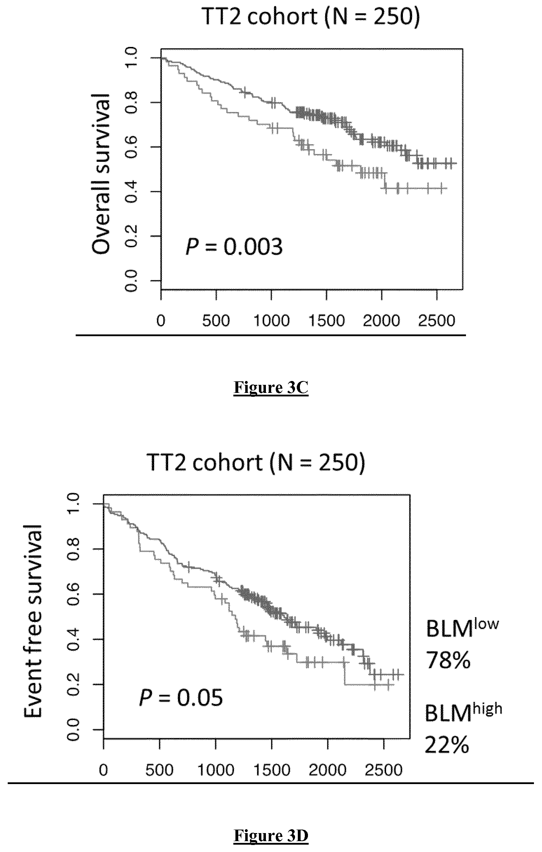

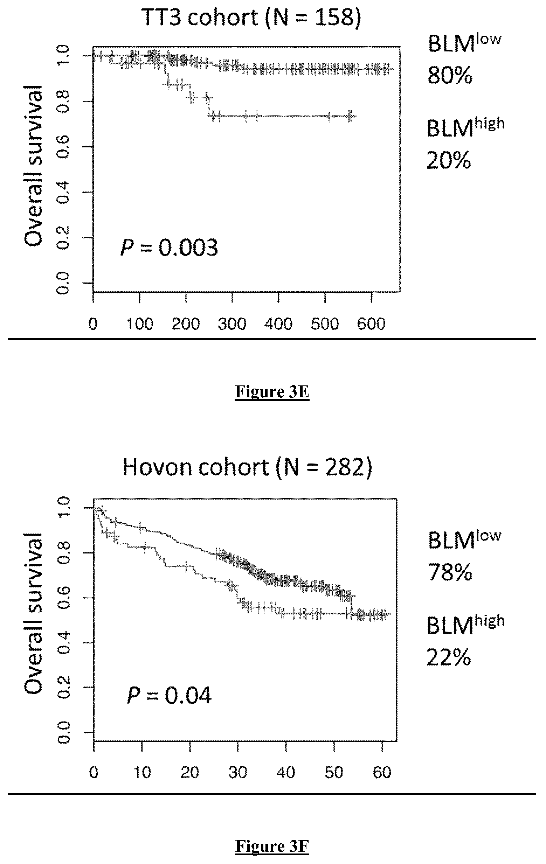

A high BLM expression, defined using maxstat R package(17), in MMCs could predict for shorter overall survival (OS) in four independent cohorts of patients (P *=* 0.003 in the HM cohort (N=206), *P* = 0.0002 in the UAMS-TT2 cohort (N=345), *P* = 0.0008 in TT3 cohort (N=186)) and *P* = 0.04 in Hovon cohort (N=282) (Figure 3). A high *BLM* expression in MMCs could also predict for shorter event free (EFS) survival in the HM and TT2 cohorts of previously-untreated patients (Figure 3).

The prognostic value of *BLM* expression was compared with conventional prognostic factors and other gene-based risk scores. In univariate COX analysis, CD-score, ISS(18), β2M, Albumin, t(4;14), del17p, HRS(19), IFM(20), GPI(21) and RS(22) have prognostic value (Table 1A). When analyzed 2 by 2, *BLM* expression tested with ISS, β2M, t(4; 14), del17p, HRS, IFM, GPI and RS remained independent in the HM cohort (Table 1B). When all parameters were tested together, BLM expression, β2M, t(4; 14) and RS score remained significant in the HM cohort (Table 1C).

Gene set enrichment analysis was performed to compare gene expression profiles of patient's MMC in high- and low-risk groups according to BLM expression using the HM cohort. Patients high BLM expression and high-risk are characterized by a significant enrichment of genes related to proliferation (REACTOME_CELL_CYCLE_MITOTIC, *P*=0.01 and REACTOME G1_S_TRANSITION, P=0.01).

**Table 1: Cox univariate and multivariate analysis of overall survival (OS) in HM patients' cohort. Table 1A**

| Univariate COX analysis | | HM-cohort | |
|---|---|---|---|
| Overall Survival | | OS | |
| | Pronostic variable | Proportional hazard ratio | *P*-value |
| | BLM | 2,94 | 0,001 |
| | ISS | 1,84 | 0,002 |
| | B2m | 1,1 | <0.0001 |
| | t(4 ;14) | 3,32 | <0.0001 |
| | del17p | 3,44 | 0,02 |
| | HRS | 2,37 | 0,01 |
| | IFM score | 2,49 | 0,01 |
| | GPI | 2,54 | <0.0001 |
| | RS | 4,16 | <0.0001 |

**Table 1B**

| 2 by 2 Multivariate COX analysis | | HM-cohort | |
|---|---|---|---|
| Overall Survival | | OS | |
| | Pronostic variable | Proportional hazard ratio | P-value |
| | BLM | 2,77 | 0,001 |
| | ISS | 1,75 | 0,005 |
| | BLM | 2,98 | 0,001 |
| | B2m | 1,1 | <0.0001 |
| | BLM | 3,1 | <0.0001 |
| | t(4 ;14) | 3,53 | <0.0001 |
| | BLM | 3,28 | <0.0001 |
| | del17p | 3,75 | 0,01 |
| | BLM | 2,84 | 0,001 |
| | HRS | 2,24 | 0,02 |
| | BLM | 2,78 | 0,001 |
| | IFM score | 2,23 | 0,04 |
| | BLM | 2,45 | 0,006 |
| | GPI | 2,27 | 0,001 |
| | BLM | 2,05 | 0,03 |
| | RS | 3,57 | <0.0001 |

**Table 1C**

| Multivariate COX analysis | | HM-cohort | |
|---|---|---|---|
| Overall Survival | | OS | |
| | Pronostic variable | Proportional hazard ratio | *P-*value |
| | BLM | 2,52 | 0,01 |
| | ISS | 1,29 | NS |
| | B2m | 1,1 | 0,02 |
| | t(4;14) | 3,12 | 0,01 |
| | del17p | 2,1 | NS |
| | HRS | 1,26 | NS |
| | IFM score | 0,42 | NS |
| | GPI | 0,78 | NS |
| | RS | 3,51 | 0,001 |

### Example 2 : BLM inhibitors as therapeutic compounds for treating individuals affected with MM.

The interest of the BLM inhibitor, ML216, to eradicate MM cells was investigated.

The effect of ML216 was investigated in 6 different human myeloma cell lines (HMCL) representative of the patients' molecular heterogeneity (Moreaux et al. Haematologica 2011). ML216 induced a dose dependent inhibition of cell growth in all investigated HMCL with a median IC50 of 4.1 µM (range: 1,2 - 30 µM) (Figure 4).

Furthermore, ML216 induced a significant apoptosis of primary myeloma cells of patients co-cultured with their bone marrow environment and recombinant IL-6(24) (n=7). BLM inhibitor significantly reduced the median number of viable myeloma cells by 54%, 60% and 74% at respectively 3, 6 and 10 µM (*P* = 0.001, *P* < 0.001 and *P* <0.001 respectively; n=7) (Figure 5A). Of interest, the non-myeloma cells present in the culture were significantly less affected by ML216 when used at 3 and 6 µM (Figure 5B). These data demonstrated a significant higher toxicity of ML216 on myeloma cells compared to normal bone marrow plasma cells.

Taken together, these data underline the therapeutic interest of BLM inhibitor in MM and especially in patients characterized by high BLM expression and a poor prognosis.

## Claims

1. The use of an expression level value of BLM in a sample obtained from an individual affected with multiple myeloma for predicting responsiveness of the said individual to a treatment of multiple myeloma.

2. An *in vitro* method for predicting the responsiveness of an individual affected with multiple myeloma to a treatment of multiple myeloma comprising the steps of:
a) measuring the expression level of BLM in a sample obtained from the said individual,
b) comparing the expression level value of BLM measured at step a) with a reference value, and
c) determining the responsiveness of the said individual to a treatment of multiple myeloma from the comparison performed at step b).

3. The *in vitro* method according to claim 2, wherein step c) comprises calculating a responsiveness score value.

4. The *in vitro* method according to any one of claims 2 and 3, wherein the expression level of BLM consists of the level of expression of the BLM gene.

5. The *in vitro* method according to any one of claims 2 and 3, wherein the expression level of BLM consists of the level of BLM protein expression.

6. The *in vitro* method according to any one of claims 1 to 5, wherein a high expression level of BLM is indicative of a bad responsiveness of the said individual to a treatment of multiple myeloma with a medicament distinct from a BLM inhibitor.

7. The *in vitro* method according to any one of claims 1 to 5, wherein a low expression level of BLM is indicative of a good responsiveness of the said individual to a treatment of multiple myeloma with a medicament distinct from a BLM inhibitor.

8. The *in vitro* method according to any one of claims 1 to 7, wherein the said sample is selected in a group comprising a bone marrow-derived sample and/or a blood-derived sample.

9. The *in vitro* method according to any one of claims 1 to 8, wherein a high expression of BLM is indicative of a good responsiveness of the said individual to a treatment with a BLM inhibitor.

10. A method for determining the efficiency of a treatment of multiple myeloma in an individual comprising the steps of;
a) performing an *in vitro* prediction method of the outcome of multiple myeloma on a sample obtained from the said individual before treatment, comprising the steps of:
a1) measuring the expression level of BLM in a sample obtained from the said individual,
a2) comparing the expression value of BLM measured at step a1) with a reference value, and
a3) determining a first prediction of outcome of multiple myeloma in the said individual from the comparison performed at step a2).
b) performing an *in vitro* prediction method of the outcome of multiple myeloma on a sample obtained from the said individual after treatment, comprising the steps of:
b1) measuring the expression level of BLM in a sample obtained from the said individual,
b2) comparing the expression value of BLM measured at step b1) with a reference value, and
b3) determining a second prediction of outcome of multiple myeloma in the said individual from the comparison performed at step b2).
c) determining if the second prediction determined at step a3) means a better outcome than the first prediction determined at step b3).

11. The *in vitro* method according to claim 10, wherein the expression level of BLM consists of the level of expression of the BLM gene.

12. The *in vitro* method according to claim 10, wherein the expression level of BLM consists of the level of BLM protein expression.

13. The *in vitro* method according to any one of claims 10 to 12, wherein the said sample is selected in a group comprising a bone marrow-derived sample and a blood-derived sample.

14. An *in vitro* method for determining the responsiveness of an individual affected with multiple myeloma to a treatment which induces an inhibition of BLM expression, comprising the steps of :
a) measuring the expression level of BLM in a sample obtained from the said individual,
b) comparing the expression value of BLM measured at step a) with a reference value, and
c) determining the responsiveness of the said individual to a treatment which induces an inhibition of BLM expression from the comparison performed at step b).

15. The *in vitro* method according to claim 14, wherein the expression level of BLM consists of the level of expression of the BLM gene.

16. The *in vitro* method according to claim 14, wherein the expression level of BLM consists of the level of BLM protein expression.

17. The *in vitro* method according to any one of claims 14 to 16, wherein the said sample is selected in a group comprising a bone marrow-derived sample and a blood-derived sample.

18. A BLM inhibitor for its use for treating an individual affected with multiple myeloma.
